Europäisches Patentamt

European Patent Office

Office européen des brevets

(11) Publication number: **0 285 987 B1**

## EUROPEAN PATENT SPECIFICATION

(45) Date of publication of patent specification: **03.02.93**

(51) Int. Cl.5: **A01N 63/04**, A01C 1/06, C12N 1/14, //(C12N1/14, C12R1:885)

(21) Application number: **88105088.4**

(22) Date of filing: **29.03.88**

(54) **Fused biocontrol agents.**

(30) Priority: **03.04.87 US 34304**

(43) Date of publication of application:
**12.10.88 Bulletin 88/41**

(45) Publication of the grant of the patent:
**03.02.93 Bulletin 93/05**

(84) Designated Contracting States:
**AT BE CH DE ES FR GB GR IT LI LU NL SE**

(56) References cited:

APPLIED AND ENVIRONMENTAL MICROBI-OLOGY, vol. 47, no. 2, February 1984 (Washington DC) H.TOYAMA et al. "Protoplast Fusion of Trichoderma reesei, Using Immature Conidia" pages 363-368

CHEMICAL ABSTRACTS, vol. 104, no. 7, February 17, 1986, Columbus, Ohio, USA S.J.GRACHECK et al. "Proto- -plast formation and fusion using Trichoderma reesei mutants" page 312, left column, Abstract-no. 48 608x

(73) Proprietor: **Cornell Research Foundation East Hill Plaza Ithaca, N.Y. 14850(US)**

(72) Inventor: **Harman, Gary E. 3986 Braewood Lane Geneva, N.Y. 14456(US)**
Inventor: **Stasz, Thomas E. 238 Nursery Avenue Geneva, N.Y. 14456(US)**
Inventor: **Weeden, Norman F. 395 White Springs Road Geneva, N.Y. 14456(US)**

(74) Representative: **Kraus, Walter, Dr. et al Patentanwälte Kraus, Weisert & Partner Thomas-Wimmer-Ring 15 W-8000 München 22(DE)**

**Description**

Biological control (biocontrol) of plant pathogens is becoming an important component of plan disease management. Biocontrol potentially offers answers to many persistent problems in agriculture, including those concerning resource limitations, nonsustainable agricultural systems and over-reliance on pesticides (Cook and Baker, 1983, Am. Phytopathol. Soc. St. Paul, MN 539 pp). Biocontrol agents are particularly attractive because they may be able to colonize and protect plant portions inaccessible to conventional chemical pesticides (e.g. plant roots in soil) (Harman and Hadar, 1983, Seed Sci. and Technol. 11:-893-906; and Ahmad and Baker, 1987, Rhizosphere competence of Trichoderma harzianum, Phytopathology (in press)). Much emphasis is now being placed on the purposeful application of specific biocontrol agents, as opposed to improving the level of naturally occurring biocontrol by environmental modification (Cook and Baker, 1983, supra). Many fungi and other microorganisms have been shown to control various plant pathogens, and a few fungal agents now are being used commercially on a limited scale in Europe to control specific pathogens, including Botrytis cinerea on grapes, Ceratocystis ulmi (Dutch elm disease) on elms, and Chondrostsereum purpureum (silver-leaf disease) on ornamental trees.

The Successful use of Trichoderma species as biocontrol agents will be greatly enhanced if improved strains are developed. Five species (species aggregates) of Trichoderma (T. hamatum, T. harzianum, T. koningii, T. polysporum and T. viride) are most important for biocontrol (Cook and Baker, 1983, supra). However, biocontrol capability, as well as numerous other desirable or essential traits, is an attribute of specific strains, rather than of particular species or genera, and is extremely variable among strains. For example, most currently utilized strains are unable to grow at either high or low temperatures favored by some plant pathogenic fungi, only a few are rhizosphere competent (Ahmad and Baker, 1987, supra; Chao et al., 1986, Phytopathology 76:60-65), and most control only a narrow range of plant pathogens.

The strains presently available were obtained by selection from naturally occurring variation. Typically, large numbers of wild isolates are screened for their ability to control specific pathogens under controlled conditions. Mutation and further selection of strains has been also employed (Papavizas, 1985, Annu. Rev Phytopathol. 23:23-54; Ahmad and Baker, 1987, supra).

Genetic recombination potentially is a much more powerful method for developing superior biological control strains than selection or mutation. Strains expressing desirable attributes can be used as parents in crosses with other strains expressing other desirable traits. By so doing, progeny with combinations of traits could be developed. Unfortunately, sexual stages are rare or lacking in most strains of Trichoderma spp., and conventional sexual crosses cannot be used to genetically manipulate these fungi.

Protoplast fusion may provide a means of genetically manipulating strains of Trichoderma spp. by initiating parasexuality (Anne and Peberdy, 1975, Arch. Microbiol. 105:201-205; Fincham et al., 1979. Fungal Genetics. 4th ed. Univ. Calif. Press, Berkeley. 636 pp). In fact, interspecific and even intergeneric crosses may be feasible. Intraspecific crosses have been accomplished in Trichoderma reesei (Gracheck, 1984, Protoplast formation, regeneration, and fusion in Trichoderma, Ph.D. Thesis, Univ. Arkansas.; Toyama et al., 1984, Appl. Environ. Microbiol. 47:363-368). Thus it may be possible to combine desirable traits from various parental strains to produce superior biocontrol strains.

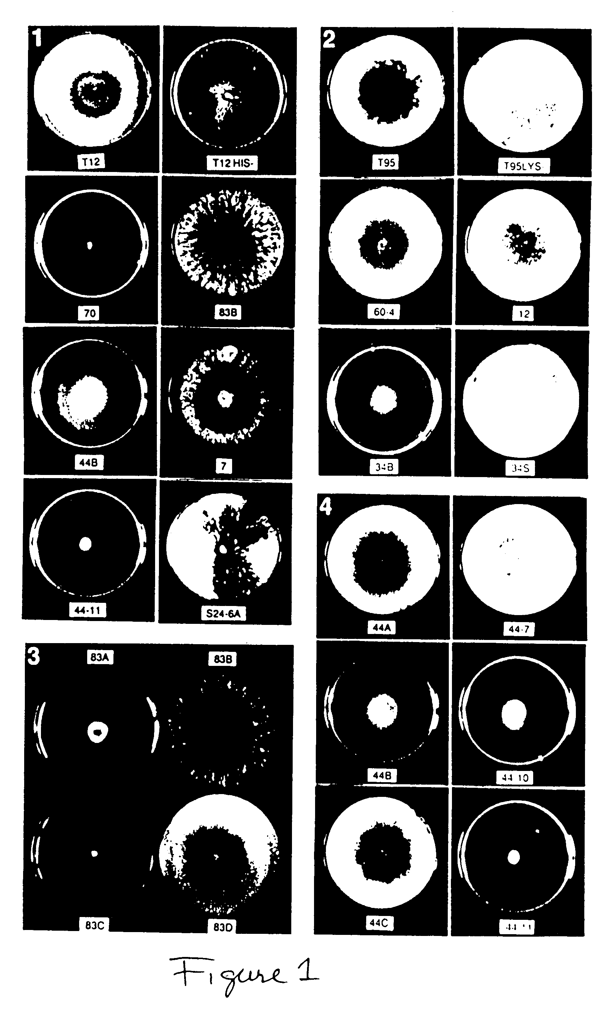

Figure 1A shows the appearance of the wild type T12, T12 his, and various progeny strains derived from fusion of Trichoderma harzianum T12 his⁻ and T95 lys⁻. All strains shown are of the T12 isozyme phenotype. Cultures were grown in petri dishes 10 cm in diameter on potato dextrose agar for 6 days.

Figure 1B shows the appearance of the wild type T95, T95 lys⁻, and various progeny strains derived from fusion of Trichoderma harzianum T12 his⁻ and T95 lys⁻. All strains shown are of the T95 isozyme phenotype. Cultures were grown in petri dishes 10 cm in diameter on potato dextrose agar for 6 days.

Figure 1C shows the variation in strains derived from sectors of strain 83. 83A, 83B, 83C are of T12 isozyme phenotype, while 83D is of T95 phenotype. Cultures were grown in petri dishes 10 cm in diameter on potato dextrose agar for 6 days.

Figure 1D shows the variation in strains derived from sectors (44A, 44B, 44C) or single spores (44-7, 44-10, 44-11) of strain 44. Strains 44A, 44C, and 44-7 are of the T95 isozyme phenotype, while 44B, 44-10, and 44-11 are of the T12 phenotype. Cultures were grown in petri dishes 8 cm in diameter on potato dextrose agar for 6 days.

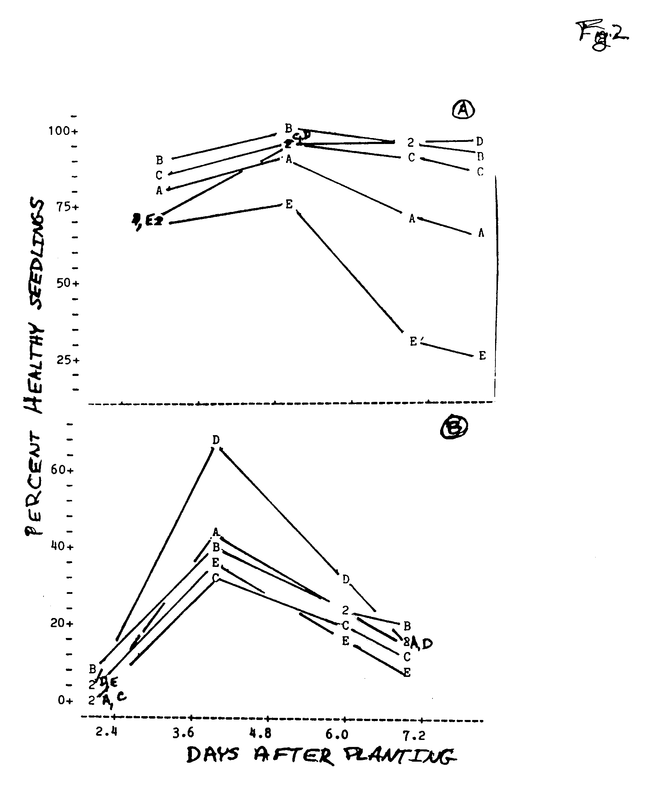

Figure 2 graphically present the numbers of healthy seedling produced after treatment of cucumber seeds with strains of Trichoderma harzianum using solid matrix priming (BA) or a conventional Methocel slurry (B) seed treatment. Strains used were the parental strain T12 (A), 1295-74 (B), 1295-7 (C). 1295-22 (D), and parental strain T95 (E). In Fig. 1A, differences at day 3 and 5 are nonsignificant, while the Minimum Significant Difference at days 7 and 8 are, 40 and 42%., respectively, according to Waller and Duncan's K-ratio test (K = 100). In Fig. 1B, differences at days 2, 6, and 7 are nonsignificant, and the minimum significant difference at day 4 = 17%.

Protoplasts from two strains of Trichoderma harzianum were fused and gave rise to numerous types of progeny strains. Rapid-growing sorts with an isozyme phenotype like that of the T12 parent are superior biocontrol agents. When cucumber seeds are treated with these strains in a solid-matrix priming system, nearly perfect stands are obtained when these seeds are planted in Pythium-infected soil. Seeds so treated produce seedlings that largely are unaffected by post-emergence damping off or root disease. Parental strains give rise to similarly large numbers of seedlings initially, but these are subject both to post-emergence damping off and to root disease. However, even seeds treated with the parental strains are better protected than if seeds are treated with the fungicide thiram. However, differences among strains largely are not seen when seeds are treated using a conventional methocel slurry. This lack of difference may result because Pythium spp. infect seeds very rapidly, and, in the methocel slurry treatment, Trichoderma spores of any strain may not germinate sufficiently rapidly to impede this early seed infection. In addition to providing excellent seed and seedling protection, one strain (1295-22) colonizes root surfaces well. This ability (rhizosphere competence) is a desirable attribute of biocontrol agent, since it allows agents to readily protect roots against attack by soil-borne fungi. The fused biocontrol agents of the invention can be used in a process wherein seeds are primed or osmoconditioned in the presence of biocontrol agents. In this process seeds in the presence of biocontrol agents of the invention, for example seeds concurrently or previously inoculated with the fused trichoderma strain biocontrol agents of the invention are primed with water using a solid phase matrix rather than using aqueous solutions.

The solid matrix comprises finely divided non plant pathogenic, water retaining solid material preferably a carbonaceous, most preferably lignateous solid which has a large equilibrium water potential $\psi$, and preferably has an osmotic potential component which is at least about 90% and preferably greater than 95% of the total water potential. Examples of such materials include coal, especially soft coal, lignateous shales such as the leonardite shale, sold as Agro-Lig, and sphagnum moss.

The solid matrix material, when containing the water necessary to prime the seeds in question, must still be sufficiently friable, nonclumping etc. so that when desired, it can be mechanically separated from the treated seeds after treatment without damage to the seeds. The particle size of the matrix material is not unduly critical, but for mechanical reasons should be smaller than seed being treated, e.g. usually less than about 20 mesh, but preferably substantially smaller. Typically a material less than about 60 mesh is preferred; for example, the Agro-Lig described hereinafter was 90% /wt less than 200 mesh; the soft coal was less than 60 mesh.

In the solid matrix priming process, the seed to be treated, an inoculum comprising a fused trichoderma strain of the invention, and a predetermined amount of water are admixed and the mixture allowed to stand, preferably in a container which allows entry of air but which reduces evaporative losses, for example a closed container with a small top opening, for a time and at a temperature sufficient to allow the seeds to imbibe water from the matrix and come to a water content equilibrium sufficient to enhance emergence, growth or yield characteristics, but short of that which would cause the seed to sprout.

As in solution priming, the equilibrium amount of water for the system to prime the seed is dependent on the specific seed variety, its state or condition , and the water potential $\psi$ of the medium, here the solid matrix material. Typically the solid matrix material should have a water potential $\psi$ between about -0.5 to about -2.0 megapascal at equilibrium with the seeds. The seed priming art to some extent is still imperical, and while typical water amounts and media water potentials for a given seed type are already generally known in the art, it is frequently best to test a small seed sample over a readily determined range of water potentials and temperatures to determine what conditions of temperature, water potential and time cause maximum imbibing of water by the seed, short of sprouting of the seed.

In the preferred process of the invention a known weight of seed is wet with water and is also or has previously been inoculated with the biocontrol agent. The wetted seed in turn, is mixed with the dry, flowable, particulate solid matrix material, coating the wet seeds with the particulate matrix material. The remainder of the predetermined amount of water for osmoconditioning is then admixed with the coated seeds and the mixture held at a predetermined temperature usually between about 10°C and about 25°C for a time sufficient to allow the seeds to reach a desired moisture content equilibrium, usually one to about fourteen days.

It has now been discovered that when the biocontrol agents described herein, i.e. strains 1295-7 (ATCC-20846), 1295-22 (ATCC 20847) and 1295-74 (ATCC 20848) are added to the solid matrix priming compositions in the conjoint process described in the pending application, they function in a significantly improved manner than when used alone. While not intending to be bound by these theories, it is presently believed that in the system of the invention the biological control agents themselves are primed or proliferate during the solid matrix priming and thus they are either more active or have more effectively

EP 0 285 987 B1

colonized the seed coat surface prior to planting. What has been observed however is that post emergence damping-off was less, or more rapid emergence occurred, or more rapid growth was observed.

The biocontrol agent can be applied to the seed as a seed coat in a known fashion prior to the solid matrix priming step.

Typically the antagonist strains can be inoculated onto the seed in any manner known in the art, but for the purposes of this invention are usually applied as a suspension in water prior to solid matrix priming. (Though they could be applied by prior colonization on the solid matrix material in sufficient numbers so that when contacted with the seed sufficient numbers of antagonist would in turn establish themselves upon and coat the seed.)

When used independently as a seed coating typically about $10^8$ conidiospores of Trichoderma spp per ml are suspended in water and sufficient volume is used to ensure complete coverage of the seed surfaces this is usually 20 to 80 ml/kg of seed, the amount varying with the weight/surface ratio of the particular seed.

When used in conjunction with the solid matrix priming step the conidiospores can be added to the water used to initially wet the seed.

The seed that can be treated can be virtually any seed that benefits from osmoconditioning including most vegetable crops, and ornamental and agronomic crops. Included are cucumbers, lettuce, carrot, onion, melon, sweet corn, tomatoes, egg plant, peppers, flower seeds and alfafa.

Alternatively, fused strains of this invention can be inoculated onto or into the seed of the plant to be protected by means of a seed treatment or coat in any manner known in the art. Generally seed treatment formulations comprise a liquid or particulate vehicle such as an inert liquid or inert dust or powder containing the active antagonist. Preferably the seed treatment or coating contains an ingredient which assists the antagonists to penetrate or stick to the seed being treated. Examples of commercially available stickers include Methocel A4C$^R$ (Dow Chemical Co.) or Pelgel$^R$(Nitragin Co.). Typically, spores or other propagules of the active antagonist are mixed with a viscous suspension of a sticker in water, and are then applied to the seed. Best results are obtained when about $10^8$ conidiospores of the Trichoderma spp. or about $10^{10}$ cells of Enterobacter cloacae are suspended in 10%(w/v) Pelgel$^R$ or 2%(w/v) Methocel$^R$. Preferably, sufficient volumes of one of these mixtures are used to ensure complete coverage of the seed surfaces. These usually require, for example, about 20 ml/kg of seed to about 80 ml/kg of seeds, the necessary amount varying with the weight-surface ratio of the particular seed.

The fused strains are employed in an amount and in a manner to prevent or retard, that is control, the attack of a soil borne pathogen on the seed being protected.

When employed in a seed drill fluid, the fluid generally comprises a solution of a water soluble polymer. The polymer when dispersed in water increases the viscosity of the solution. The viscous solution or gel serves to suspend seeds, protects hydrated/germinated seeds from mechanical damage during sowing and acts as a lubricant to facilitate seed movement in the planter.

Polymers which can be employed to prepare seed drill fluids include hydroxyethyl cellulose, synthetic magnesium silicate clays, polyacrylamide and starch graft polymers other polymers known in the art may also be employed in a manner known in the art. Those polymers which are non-ionic are best suited as a carrier for the biological control organisms of the invention. Non-ionic gels are stable over a wide pH range, have little pH buffering and are generally stable with the addition of organic or unorganic chemicals.

The amount of the fused strain typically employed in a seed drill fluid is generally at least about $10^8$ spores/ml.

Although not presently recommended, if desired the antagonists of this invention could be broadcast or furrow applied.

If desired, the seeds and/or the priming mixture can be pH adjusted for maximum biocontrol agent effectiveness.

Example 1

Media. Several media were used. These included the basal medium (BM) of Toyama, 1984, supra, which contained 2.8 g/l $(NH_4)_2SO_4$, 600 mg/l urea, 4 g/l $KH_2PO_4$, 600 mg/l $CaCl_2$ $2H_2O$, 40 g glucose, 200 mg/l $MgSO_4$, 10 mg/l $FeSO_4$ $7H_2O$, 2.8 mg/l $ZnSO_4$ $H_2O$, 3.2 mg/l $MnSO_4$ $H_2O$, 4 mg/l $CoCl$ $6H_2O$, and 20 g agar. All components except the agar were dissolved water at a 2x concentration, and filter-sterilized by passage through a 0.45 $\mu$m filter. The agar was sterilized by autoclaving the two components. They were then brought to 60°C, mixed, and poured into petri dishes. Addition of 10% w/v sucrose as an osmiticant resulted in protoplast regeneration medium (PRM). The media amended with either 150 $\mu$g/ml histidine, 150 $\mu$g/ml leucine, or the combination, and were designated as BM+L, BM+H, or PRM+L, PRM+H, or

4

PRM + HL for the basal or the protoplast regeneration medium plus these amino acids, respectively.

In addition Difco potato dextrose agar and broth (PDA and PDB, respectively) were used, as well as PDA amended with amino acids and/or 10 $\mu$g/ml benomyl, to give PDA + H, PDA + L, PDA + HL, or PDA + HLB. Benomyl was suspended in 10 ml sterile water and added to sterile medium cooled to 60°C, prior to pouring.

Strains used. Parental strains of Trichoderma harzianum Rifai were T95 (Ahmad and Baker, 1986, supra; American Type Culture Collection [ATCC] 60850) and T12m (Hadar et al., 1984, Phytopathology 74: 106-110; ATCC 20737). T95 was a mutant resistant to benomyl at 50 $\mu$g/ml (ben[+]), while growth of T12 was prevented by 3 $\mu$g/ml benomyl (ben[−]). Auxotrophic mutants requiring lysine or histidine (T95 lys[−], T95 his[−], and T12 his[−]) were prepared by irradiating conidia with ultraviolet irradiation from a 15 watt germicidal fluorescent lamp until ca. 99% of the conidia were killed. Irradiated conidia were transferred to a broth prepared from BM, but without agar. This mixture was incubated with shaking at 25°C for 1 week and was filtered daily through four layers of sterile cheesecloth to remove germinated prototrophic conidia. The nongerminated conidia were then plated on BM + HL containing 0.1% w/v Igepal Co630 as a colony restrictor (Norton and Harman, 1985, Can. J. bot. 63:1040-1045), and incubated at 25°C until colonies developed. These colonies were then individually transferred back to BM, and colonies that did not grow on this medium tentatively were considered to be auxotrophs. Identity of auxotrophs were confirmed by inability to grow on BM, but ability to grow on BM + H or BM + L. Once auxotrophs were found, they were single-spored to obtain homogenous and stable auxotrophs. Only auxotrophs that gave no growth upon repeated transfers to BM from actively-growing colonies BM + H or BM + L were used further. Plating of conidia from auxotrophs gave reversion frequencies of one in $10^{10}$ or less.

Isozyme analysis. Additional genetic markers were identified by subjecting extracts to horizontal starch gel electrophoresis followed by specific enzyme stains. For this purpose, cultures were grown in 10 ml of PDB in 25 ml flasks for 3-5 days at 25°C on a reciprocating shaker. Resulting thalli (appx. 50 mg dry weight) were removed from flasks, dried briefly on filter paper, and placed in about 0.2 ml ice-cold extraction buffer (0.05M tris (hydroxymethyl) aminomethane - HCl pH 7.1). Two gel buffer systems were used for the analysis, these were the histidine gel system at pH 6.5 described by Cardy et al., 1972. Techniques for starch gel electrophoresis of enzymes from maize (Zea mays L.). Dept. of Statistics Mimeo Series No. 1317, North Carolina State University, Raleigh, N.C., and the tris citrate/-lithium borate system of Selander et al., 1971. Univ. Texas Publ. 7103:49-90. The procedure for electrophoretic analysis of the extracts was described by Weeden, 1984, Euphytica 33: 199-208. Strains T12 and T95 were screened for reproducible differences in isozymic mobility on about 70 enzyme systems, using the procedures described by Weeden, 1984, supra. Of these, four were found to give reproducible and clearly resolved differences between strains T95 and T12. These enzymes were fumarase (FUM, E.C. 4.2.1.2.), phosphoglucomutase (PGM, E.C. 2.75.1) $\alpha$-D-glucosidase (GLU, E.C. 3.2.1.20), and triosephosphate isomerase (TPI, E.C. 5.3.1.1). The histidine gel system was used for FUM, PGM, and GLU, while the tris-citrate/lithium borate system was used with TPI. Visualization of enzyme activity was accomplished using the assay systems described by Weeden and Gottlieb 1980, Plant Physiol. 66:400-403 for PGM and TPI, except that assay mixtures were applied as agar overlays. The method of Brewer 1970. An Introduction to Isozyme Techniques. Academic Press, New York, NY. 186 pp was used for FUM. A modification of the assay described by Smith 1976. Starch gel electrophoresis. In Chromatographic and Electrophoretic Techniques. Vol II, Yearbook Medical Publishers Inc. Chicago, pp 153-209 that contained 0.1 M tris-HCl pH 7.1 and 3 mM 4-methylumbelliferyl $\alpha$-D-glucoside was used for GLU.

Protoplast isolation. Various procedures were evaluated to obtain high yields of viable protoplasts. We attempted to isolate protoplasts from immature conidia (Toyama et al., 1984, supra), from germinating conidia, and from young nonsporulating thalli.

Immature (nonpigmented) conidia were harvested from the advancing edge of colonies on PDA, suspended in sterile water and filtered through lens paper to free the conidia of hyphal debris. Conidial suspensions were mixed with 10% $\beta$-glucuronidase type H2, 10 mg/ml drieselase, 25 $\mu$g/ml chitase (Sigma Chemicals, St. Louis, MO) and 0.6 M sucrose (Stasz and Harman, 1985, Phytopathology 75:1327), and incubated at 30°C. To isolate protoplasts from germinated conidia, mature conidia were placed in 200 ml of PDB amended with 1.5% w/v yeast extract and incubated for 16 hours 25°C with shaking. Germinated conidia were collected by filtration onto Miracloth (Calbiochem, La Jolla, CA) in a Buchner funnel, and appx. $10^6$-$10^7$ were asceptically transferred either to 20 ml of the enzyme mixture described above, or to a mixture containing 13 mg/ml NovoZym 234 (Novo Laboratories, Wilton, CT) in 0.7 M NaCl, and incubated for 24 hours at 30°C with gentle shaking. Finally, protoplast isolation from young mycelium was tested by transferring appx. 100 small squares (2-4 mm$^2$) from young, nonsporulating colonies on PDA to 200 ml of the PDB plus yeast extract medium described above, and incubating these overnight with shaking at 25°C to give spherical thalli, each about 4-6 mm in diameter. These were collected on Miracloth and incubated 24 hours in 80 ml of the NovoZym-NaCl mixture described above.

In all cases, protoplast-enzyme mixtures were filtered aseptically through 4 layers of cheesecloth to remove hyphae and other debris, and protoplasts were harvested by centrifugation at 100xg for 5 minutes. Protoplasts were resuspended and washed in a variety of media, including PBS (0.01 NaPO$_4$ pH 7.0 plus 0.7 M NaCl) or STC (0.6 M sorbitol, 0.01 M tris-HCl, and 0.01 M CaCl$_2$ all at pH 7.5) (modified from Turgeon et al., 1985, Molec. Gen. Genet. 201:450-453). A number of inorganic salts were substituted for the sorbitol osmoticant in STC, including 0.7 M NaCl, 0.6 M M$_9$SO$_4$, 1.2 M M$_9$SO$_4$, 0.6 M KCl, 0.4 M (NH$_4$)$_2$SO$_4$, 0.6 M mannitol, and 0.6 M sucrose.

Numbers of viable protoplasts in suspensions were determined by preparing stepwise dilutions in one of the suspension media (usually STC) described above, and then plating on PRM. Colonies typically developed from protoplasts with 48 hours of plating at 25°C. Protoplasts were distinguished from spores and hyphal fragments by preparing similar dilution series in distilled water, followed by plating on PRM. Protoplasts rupture upon exposure to water, while walled propagules do not. In all cases, numbers of colonies developing on PRM were compared with total numbers of protoplasts as determined by counts in a Petroff Hausser bacterial counting chamber (Thomas Scientific, Swedesboro NJ).

Microscopic observation of nuclei Determination of the number of nucli present in various cells was accomplished by fixing cells for 30 minutes in 3% formaldehyde dissolved in 50 mM sodium phosphate buffer at pH 7. Cells were then washed twice in the phosphate buffer, stained by adding 0.1 g/ml 4'-6-diamidino-2-phenyldihydrochloride (DAPI), and then rinsed twice again with phosphate buffer. Specimens so prepared were viewed using epiflourescent illumination on a Bausch and Lomb microscope equipped with an exciter filter passing light between 300 and 400 nm, a dichroic reflector reflecting light below 450 mm, and a barrier filter passing light above 475 nm. DAPI has a high specificity for staining DNA (Shapiro, 1985. Practical Flow Cytometry. Alan R. Liss, Inc., New York, N.Y. 295 pp).

Protoplast Fusion. Protoplasts were fused using a procedure similar to that described by Turgeon et al., 1985, supra. One ml of a suspension containing about 10$^8$ protoplasts in STC were prepared with about that contained equal numbers of protoplasts from each parental strain. To this was added 200 $\mu$l of solution containing 60% (w/v) polyethylene glycol (PEG) solution (appx. molecular weight 3350, Sigma Chemical Co., St. Louis, MO), 10 mM CaCl$_2$, and 10 mM tris-HCl, pH 7.5. The PEG was mixed with the protoplast suspension by gently rolling the tube. A second 500 $\mu$l aliquot of the PEG solution was added, the mixture again gently mixed, and finally a third 500 $\mu$l aliquot was added and mixed by rolling. Following incubation at 30°C for 10 minutes, the mixture was then diluted with 1.1 ml of STC, the mixture mixed gently, This dilution step was repeated, and finally 2.2 ml of STC were added. After fusion and dilution protoplasts were recovered by centrifugation and resuspended in 5 ml STC.

Protoplasts from T95 his$^-$ with protoplasts from T95 lys$^-$ and T12 his$^-$ with T95 lys$^-$ were fused. As checks for possible reversion, each parental line was fused with itself. Protoplast suspensions were serially diluted in STC or in water and then plated on PRM. Serial dilutions from all fusions were plated on PRM+HL to determine the total number of viable protoplasts in each experiment.

Analyses of Progeny. After fusion of T95 lys$^-$ with T95 his$^-$, thalli developing on PRM were transferred to BM to eliminate the possibility of cross-feeding that may have occurred when heavy protoplast suspensions were applied to plates. Whether prototrophic progeny were heterokaryotic or had resulted from nuclear fusion was tested. Because each conidium receives only a single nucleus from the conidiophore (Picataggio et al., 1984, Eur. J. Appl. Microbiol. Biotechnol. 17;121-128; Toyama et al., 1984, supra) dissimilar nuclei segregate during conidiation. Conidia from progeny strains were suspended in water, filtered through a 5 $\mu$m pore size filter (Acrodiscs, VWR, Rochester, NY) to remove hyphal fragments and dilutions were plated on BM, BM+H, BM+L, and BM+HL. In order to determine whether colonies that developed on BM were diploids, or whether they were derived from a conidium or other propagule containing two nuclear types, conidia were again isolated from these strains, filtered, and again plated on the array of media noted above.

With the T12 his$^-$ plus T95 lys$^-$ cross, a more complete genetic analysis could be conducted. Thalli developing on PRM were transferred to BM to eliminate the possibility of cross-feeding that may have occurred when heavy protoplast suspensions were applied to plates. After colonies reached a diameter of >5 mm in diameter, portions were transferred to 10 ml PDB in 25 ml flasks for isozyme analysis, and to BM, BM+H, BM+L, BM+HL, PDA+HL, and PDA+HLB. Additionally, conidia were harvested, filtered through a 5 $\mu$m filter, and plated on the media noted above. Numbers of colonies and appearance of colonies derived from a single conidia were noted, and representatives of each colony type were tested on the isozyme assay system. In addition, sectors were frequently noted in colonies of progeny from this cross. These were analyzed by their ability to grow on the media noted above and by their isozyme phenotypes.

RESULTS

Protoplast preparation. Initial attempts to prepare protoplasts were conducted with immature or mature conidia. Enzyme preparations tested included the driesalase, glucuronidase, and chitinase mixture described above, the mixture modified to contain five-fold more chitinase, each of the enzymes alone, and NovoZym 234. Conidia were treated for up to 6 hours, were then placed in either water of STC, and numbers of cells were counted in a Petroff-Hausser microbiological counting chamber to determine numbers of osmotically-sensitive or insensitive cells. In no case were preparation obtained with more than 50% osmotically-sensitive cells (protoplasts). The most effective enzyme preparation was the mixture containing five-fold additional chitinase. Individual enzymes (chitinase, driesalase, $\beta$-glucuronidase, or NovoZym 234) gave fewer than 10% osmotically-sensitive cells.

Since protoplast yields were low with conidial suspensions, the liberation of protoplasts from young thalli or conidial germlings was investigated using Grachek's (1984) procedure. Either germlings or young thalli gave high yields (appx. $10^8$/flask) of protoplasts. At least 99% of these were osmotically sensitive; in some batches 99.99% were sensitive, as measured by growth on PRM after aliquots were diluted either in water or in STC. On PRM, visible thalli developed after 48 hours inocultation at 25°C. Protoplasts were released more rapidly from T95 than from T12; equivalent numbers of protoplasts were released from T95 after 90 minutes incubation as were released from T12 after 180 minutes.

Viability of protoplasts were determined by comparisons of counts of protoplasts in Petroff-Hausser chambers with plating on PRM. When protoplasts were suspended in STC, or in the similar mixtures but with various inorganic salts or sugars substituted for 0.6M sorbitol, about 10% of the protoplasts formed thalli. If however, PBS was used as the suspension medium, protoplasts did not regenerate. In all further work, STC was used as the suspension medium, and all protoplasts were prepared from young thalli.

Numbers of nuclei per propagule. Two to twelve nuclei were observed in protoplasts prepared from thalli, from germlings, or from immature conidia. Similar numbers of nuclei were observed in conidia.

Fusion between T95 his⁻ and T95 lys⁻. As a test of procedures developed, protoplasts of two auxotrophs of the same parental strain were fused. Fusion occurred between two cells, but aggregates of several cells were formed more frequently. Fused protoplasts were plated on PRM + HL or on PRM. On PRM + HL, $5 \times 10^5$ thalli arose from the fused protoplast mixture, while on PRM, $5 \times 10^4$ colonies developed, so the fusion frequency was about 10%. As a control T95 lys⁻ was fused with itself, as was T95 his⁻, and these also were plated on PRM or PRM + HL. On PRM, no colonies developed, while on PRM + HL, $28 \times 10^4$ and $10 \times 10^5$ colonies developed from T95 lys⁻ and T95 his⁻, respectively. On all media, visible thalli developed after 48 hour incubation. Fusion aggregates of two to many cells all gave rise to thalli, as determined by microscopic observation.

These results demonstrate that the colonies that grew on PRM after fusion between T95 lys⁻ and T95 his⁻ are prototrophic as a consequence of complementation between the fused parental strains. However, complementation may be due either to heterokaryosis or as a consequence of karyogamy. Therefore, conidial suspensions from twelve prototrophic progeny from this cross were diluted and plated on PRM, PRM + H, PRM + L, or PRM + HL. Dilutions on PRM + L, PRM + H, or PRM + HL gave $10^4$-$10^5$ thalli, while on PRM alone 0-2 thalli were recovered. These few prototrophic strains were allowed to sporulate, and conidia from these plated on the various media, and again, we obtained only about 1 prototrophic thallus per $10^5$-$10^6$ that were plated. This experiment was repeated twice with similar results.

Fusion between T95 lys⁻ and T12 his⁻. Strains T95 lys⁻ and T12 his⁻ were fused in two separate experiments. In both cases, thalli developed on PRM very slowly. The first thalli became visible after 1-2 weeks incubation, and even 5 weeks after fusion, new thalli were developing. These thalli were transferred to fresh BM, and upon development of a visible thallus, were assigned a number. From the first fusion, about 100 strains were harvested, while from the second about 20 were obtained and investigated further. These were tested for their isozyme patterns, and except for two (strains 12 and 34) all were identical to the pattern of the T12 parent. Additive combinations of bands indicative of heterozygotes was not observed for any strain.

As cultures were incubated on BM, more rapid-growth sectors arose. These were transferred to fresh BM plates and were designated with a letter designation, e.g. strain 44A or 44B for two sectors of strain 44 differing in growth rates or colony morphology. Some of these, when subjected to electrophoresis, gave isozyme patterns like those of the T12 parent and some were like that of T95. Again, double banding patterns were not observed.

Colony morphology and growth rates of these progeny strains were extremely variable. Two strains (7 and 22) eventually gave rise to cultures growing more rapidly than either parent on BM, others grew as fast as the original parents, while still others grew much more slowly (Fig. 1A). Colony morphology ranged from small, wrinkled, dark-brown pigmented strains with very sparse sporulation (e.g. strain 83A in Fig. 1A) to ones that resembled one or the other wild-type parents (strains 12 and 7 in Fig. 1A and 2, Table 1). Figure 1A shows a range of colony morphologies and growth of strains with T12 enzyme phenotype on a permissive medium (PDA) relative to the wild-type and auxotrophic T12 parents. Figure 1B shows a similar assay among strains with the T95 enzyme phenotype.

Strains were grown on a range of media (BM, BM + H, BM + L, BM + HL, DA, PDA + HLB) to define their nutritional requirements and resistance to benomyl. These data are summarized in Table 1 for selected strains, along with their colony morphologies. All strains originally grew slowly on BM, but some sectored to give rise to fast-growing, fully prototrophic strains (e.g. 7, 12, 44A and several others). Others were very different in colony morphology than either parent (Fig. 1A, Table 1). Some (e.g. 34, 83A, B, or C) were not recognizable as Trichoderma spp.; however their enzyme phenotype matched that of one or the other parental type.

Similarly, variation was noted when single spores were plated on the various media and distinct colony morphologies could be selected. Strain 24, for example, gave rise to at least three distinct categories of sub-progeny, i.e. a weakly prototrophic white colony that sporulated very little (Table 1), a type similar in all respects to the original T12 his$^-$, and a rare type (appx 1 in $10^5$) that was similar to T95 in all respects, including isozyme phenotype. Two other strains similar in all respects to T12 his$^-$ produced conidia that gave rise to subprogeny similar in all respects to T95.

As a consequence of sectoring and single-spore isolations, families of progeny strains were obtained that arose from the same original thallus, but which differ markedly in all characteristics measured. Examples of these are given in Figs. 1E and 1D. Sectors from strain 83 (Fig. 1C) gave rise to slow-growing, wrinkled, nearly asporulent brown strains (83A or C) (the pigmentation became more intense as incubation was prolonged) and a more rapid-growing pinnate strain (83B), all of which have the T12 isozyme phenotype. Strain 83D, conversely, grew rapidly, sporulated well and was of the T95 isozyme phenotype. These characteristics were transmitted through conidiation (Table 1). Strain 44 originally was a slow-growing strain with the T12 phenotype, while sectors derived from it were similar in all respects to the rapid-growing wild-type T95 parent (strains 44A and C in Fig. 1D) or a moderately-slow growing yellow-brown strain with the T12 phenotype (44C in Fig. 1D). Similarly, colonies derived from single spores differed markedly; strain 44-7 was again similar to the wild-type T95 parental strain, while 44-10 and 44-11 were slow-growing strains with the T12 phenotype. All of the strains in Figs. 1C and 1D were prototrophic except strain 44-11, which required histidine for growth.

TABLE 1. Colony morphology, enzyme ph; :otype, and growth rate on var:.us media of selected progeny strains resulting from fusion of auxotrophs of strains T12 and T95 of <u>Trichoderma harzianum</u>. Also presented are data on the range of single-spore sub-progeny that were obtained from these strains.

| Strain | Enzyme phenotype | Colony appearance | Growth on various media | | | | | Single-spore sub-progeny types |
|---|---|---|---|---|---|---|---|---|
| | | | BM | BM+H | BM+L | BM+HL | PDA+HLB | |
| T12 | T12 | green-sporulation, heavy growth | F | F | F | F | - | all like original |
| T95 | T95 | light green sporulation, heavy growth | F | F | F | F | F | all like original |
| T12 his⁻ | T12 | weak sporulation moderate growth | - | M | - | M | - | all like original |
| T95 lys⁻ | T95 | weak sporulation moderate growth | - | - | M | M | M | all like original |
| 7 | T12 | like T12 | F | F | F | F | - | all like strain 7 |
| 12 | T95 | like T95 | F | F | F | F | F | mixture of types; some like T95 lys⁻, and others like T95 |
| 24 | T12 | like T12 his⁻ | - | M | - | M | - | variety of types; including ones like T95, nearly asporulant strains (Fig. 2) with weak protrophy, and others like T12 his⁻ |
| 29 | T12 | like T12 his⁻ | - | M | - | M | - | variety of types; including stellate growth patterns (Fig. 2) |

EP 0 285 987 B1

TABLE 1. (cont...) Colony morphology, enzyme phenotype, and growth rate on various media of selected progeny strains resulting from fusion of auxotrophs of strains T12 and T95 of _Trichoderma harzianum_. Also presented are data on the range of single-spore sub-progeny that were obtained from these strains.

| Strain | Enzyme phenotype | Colony appearance | Growth on various media | | | | | Single-spore sub-progeny types |
|---|---|---|---|---|---|---|---|---|
| | | | BM | BM+H | BM+L | BM+HL | PDA+HLB | |
| 34 | T95 | very sparse growth little sporulation | S | S | S | S | S | all like strain 34 |
| 44A | T95 | similar to T95 | F | F | F | F | F | all like T95 |
| 44B | T12 | similar to T12 his⁻ | M | M | S | M | – | Two types of colonies- one type was rapid-growing, prototrophic, the other grew more and required lysine |
| 60 | T95 | like T95 | F | F | F | F | F | Two types of colonies- approximately 1/2 like T95 and 1/2 like T12 |
| 70 | T12 | very slow growth | S | S | – | S | – | all like strain 70 |
| 83A, B, or C | T12 | brown, wrinkled, very few spores (83A,C); or pinnate, sporulating (83B) | S | M | S | M | – | all like strain 83A, B, or C |
| 83D | T95 | like T95 | F | F | F | F | F | all like strain 83D |

[a] Abbreviations for media are BM, basal medium; BM+H, basal medium + histidine; BM+L, basal medium + lysine; BM+HL, basal medium + histidine and lysine; PDA+HLB, potato dextros agar + histidine, lysine, and benomyl. Abbreviations for growth rate are f, fast (35 mm diameter colonies in petri dishes after 3 days growth at 25°C; m, medium (15-35 mm growth), S, 2-15 mm growth in three days, and –, no growth.

High yields of nearly pure protoplasts can be obtained either from young thalli or germlings of T. harzianum strains T12 or T95, using a procedure similar to that used by Grachek, 1984, supra. Various enzyme preparations were used with immature or mature conidia, but none gave more than about 50% protoplasts. These results are at variance with those of Toyama et al., 1984, supra, but he used other strains and enzyme preparations. About 10% of the protoplasts from young germlings or thalli gave rise to colonies on PRM. In other systems, regeneration frequencies ranged from 1 to 50% in filamentous fungi, while regeneration of protoplasts from yeasts may approach 100% (Picataggio et al., 1985, supra; Peberdy, 1979, Ann. Rev. Microbiol. 33:21-39).

The procedure for protoplasts fusion with PEG gave high levels of fusion; with the T95 his⁻ x T95 lys⁻ cross, about 10% of the regenerating thalli were protrotrophic. All of the numerous prototrophic thalli from the cross between the two auxotrophs of T95 appeared to be stable heterokaryons. Colonies derived from single conidia of prototrophic progeny were auxotrophic for either histidine or lysine in approximately equal numbers. These data indicate that the two nuclear types were present in approximately equal numbers.

Conidia of T. harzianum are multinucleate, unlike the condition reported by Toyama et al., 1984, supra for T. reesei. There is, therefore, no advantage to preparing protoplasts from conidia; in our studies protoplasts derived from any source contain 2-12 nuclei.

Conidia are, however, apparently genetically homogenous, probably because all of the nuclei in a conidium are derived from a single nucleus (Picataggio et al., 1984, supra). Evidence for this was provided by the consistent isolation only of auxotrophs from prototrophic strains derived from fusion of T95 lys⁻ and T95 his⁻. If nuclei in conidia were derived from two or more nuclei from the thallus, prototrophic progeny should have been isolated. The very low (1 in $10^5$) rate of isolation of prototrophic strains from conidial suspensions may have resulted from a rare bit of heterokaryotic hyphae in the suspension, or from a rare heterokaryotic conidium. They do not represent stable diploids, since a second generation of conidial suspensions obtained from prototrophs derived from a single conidial suspensions again gave rise primarily to auxotrophic strains, with again only a very low level of prototrophic colonies. These results are similar to those of Picataggio et al., 1984, supra with T. reesei, and are at variance with those of Toyama et al., 1984, supra with the same species.

Events following fusion of T12 his⁻ and T95 lys⁻ followed a very different pattern. Fusion mixes with both crosses were similar in appearance. However, while regeneration of fused protoplasts of the two auxotrophs of T95 gave rise to numerous prototrophic thalli within 48 hours, fusion products of the T95 x T12 cross grew very slowly. Isozymic analysis the immediate progeny of the T12 x T95 fusion showed only the pattern of T12 in >99% of the strains. These strains also grew faster on media containing histidine than on media not containing this amino acid, and were susceptible to benomyl, all of which are characteristic of T12.

However, with increasing time of culture, more rapidly growing sectors frequently appeared. These were of both the T95 and the T12 type, indicating that both nuclear types were present in the original thallus, even though the T95 phenotype could not be detected. The weakly prototrophic nature of the original thalli from this fusion suggests that the T95 genome was present and providing a low level of nuclear complementation. Nonexpression of one parental genome has been noted in fusion products of Bacillus subtilus; with this bacterium colonies were obtained which readily switched from expression the genome of one parent to the genome of the second parent, indicating complete repression of one genome in the presence of the other (Hotchkiss and Gabor, 1980, Proc Natl. Acad. Sci. 77:3553-3557).

The results were corroborated by the behaviour of single conidial strains. Some of these were of the rapid-growing T95 phenotype even though the original progeny strains were slow-growing and of T12 phenotype. Conidia giving rise to the T95 isozyme phenotype were rare. The age of the strain (reflecting time for genetic segregation) may affect whether conidial strains will all be alike or different. Most of the progeny strains exhibiting diversity among single spores isolates (e.g. strains 24 and 29) were from the second cross, and so had been maintained only for about 4 months prior to conidial plating, while strains with no diversity (e.g., 7, 12, 34, 44A, 44B, 70, and 83A, B, C, or D) were all from the first cross, and had been maintained for 10 months prior to conidial isolation. Strain 60 however, was from the first cross and contained a range of genetic types in conidial isolations.

Some progeny strains were different from the parental strains, and maintained this nonparental character upon single spore isolation. Strain 83A, B, or C for example, had an enzyme phenotype like T12, but differed in hyphal color (brown vs. hyaline), growth type (restricted, wrinkled vs spreading, smooth), and sporulation ability (very sparse vs abundant) relative to the parental strains (Fig. 1A). Strain 34, conversely, grew very sparsely, and sporulates infrequently and has a enzyme genotype like that of T95. All conidial subprogeny of strains 83A, B, C, or D, or 34 were protrophic, and maintained all morphological and isozyme characters of the original progeny strain through conidiation. Strains 22 and 7 grow more rapidly than either the auxotrophic of wild-type parental strains. These data suggest that these some progeny are significantly different genetically from either parent.

Moreover, strains such as S24, 29, and the original strain 44, gave a variety of colony types when single conidia were isolated. These data indicate that there are a range of nuclear types in the original thallus, including ones similar to the parental strains and others that are significantly different.

Example 2

The purpose of this Example is to describe the preparation and properties of several superior biocontrol strains of Trichoderma harzianum.

Media. Several media were used. These included the basic medium (BM) of Toyama, 1984, which contained 1.4 g/l (NH$_4$)$_2$SO$_4$, 30 mg/l urea, 2 g/l KH$_2$PO$_4$, 20 g glucose, 30 mg/l MgSO$_4$, 5 mg/l FeSO$_4$ 7H$_2$O, 1.4 mg/l ZnSO$_4$ 7H$_2$O, 1.6 mg/l MnSO$_4$ H$_2$O, 2 mg/l CoCl H$_2$O, and 20 g agar. All components except the agar were dissolved water at a 2x concentration, and filter-sterilized by passage through a 0.45 μm filter. The agar was sterilized by autoclaving the two components. They were then brought to 60°C, mixed, and poured into petri dishes. Addition of 10% w/v sucrose as an osmiticant resulted in protoplast regeneration medium (PRM). The media amended with either 150 μg/ml histidine, 150 μg/ml leucine, or the combination, and were designated as BM + L, BM + H, BM + HL, or PRM + L, PRM + H, or PRM + HL for the basal or the protoplast regeneration medium plus these amino acids, respectively.

In addition, Difco potato dextrose agar or both (PDA or PDB, respectively) was used, as well as PDA amended with amino acids and/or 10 g/ml benomyl, to give PDA + H, PDA + L, PDA + HL, or PDA + HLB. Benomyl was suspended in 10 ml sterile water and added prior to sterile medium cooled to 60°C, and then poured into petri dishes.

Strains used. Parental strains of Trichoderma harzianum Rifai were T95 (Ahmad and Baker, 1987, supra; American Type Culture Collection [ATCC] 60850) and T12 m (Hadar et al, 1984, supra ATCC 20737). T95 was a mutant resistant to benomyl at 50 μg/ml, while growth of T12 was prevented by 3μg/ml benomyl. Auxotrophic mutants requiring lysine or histidine (T95) or histidine (T12) were prepared by irradiating conidia with ultraviolet irradiation from a 15 watt germicidal florescent lamp until ca. 99% of the conidia were killed. Irradiated conidia were transferred to a broth prepared from BM, but without agar. This mixture was incubated with shaking at 25°C for 1 week, and was filtered daily through four layers of sterile cheesecloth to remove protorophic conidia. The nongerminated conidia were plated on BM + HL containing 0.1% w/v Igepal Co630 as a colony restrictor (Norton and Harman, 1985, supra), and incubated at 25°C until colonies developed. These colonies were then individually transferred back to BM, and colonies that did not grow on this medium tentatively was considered to be auxotrophs. Identity of auxotrophs were confirmed by inability to grow on BM, but ability to grow on BM + H or BM + L. Once auxotrophs were found, they were single-spored to obtain homogenous and stable auxotrophs. Only auxotrophs that gave no growth upon repeated transfers to BM from actively-growing colonies BM + H or BM + L were used further. Plating of conidia from auxotrophs gave reversion frequencies of one in 10$^{10}$ or less. Colonies with a requirement for lysine are indicated by lys$^-$, for histidine by his$^-$, and benomyl resistance is indicated by ben$^+$.

Isozyme analysis. Additional genetic markers were identified by subjecting extracts of horizontal starch gel electrophoresis followed by specific enzyme stains. For this purpose, cultures were grown in 10 ml of PDB in 25 ml flasks for 3-5 days at 25°C on a reciprocating shaker. Resulting thalli (appx. 50 mg dry weight) were removed from flasks, dried briefly on filter paper, and placed in about 0.2 ml ice-cold extraction buffer (0.05M tris (hydroxymethyl) aminomethane - HCl pH 7.1). Two gel buffer systems were used for the analysis, these were the histidine gel system at pH 6.5 described by Cardy et al., 1972, supra and the tris citrate/lithium borate system of Selander et al., supra. The procedure for electrophoretic analysis of the extracts was described by Weeden, 1984, supra. Strains T12 and T95 were screened for reproducible differences is isozymic mobility on about 70 enzyme systems, using the procedures described by Weeden (1984). Of these, four were found to give reproducible and easily-distinguished differences between strains T95 and T12. These enzymes were fumarase (FUM. E.C. 4.2.1.2.), phosphoglucomutase (PGM, E.C. 2.75.1) α-D-glucosidase (GLU. E.C. 3.2.1.20), and triosephosphate isomerase (TPI, E.C. 5.3.1.1). The histidine gel system was used for FUM, PGM, and GLU, while the tris-citrate/lithium borate system was used with TPI. Visualization of enzyme activity was accomplished using the assay systems described by Weeden and Gottlieb, 1980, supra for PGM and TPI, and by Brewer, 1970, supra for FUM. A modification of the assay described by Smith, 1976, supra that contained 0.1 M tris-HCl pH 7.1 and 3 mM 4-methylumbelliferyl α-D-glucoside was used for GLU.

Protoplast isolation. Appx. 100 small squares (2-4 mm$^2$) from young, nonsporulating colonies on PDA were transferred to 200 ml of PDB amended with 1.5% yeast extract and incubated overnight with shaking at 25°C to give spherical thalli each about 4-6 mm in diameter. These were collected on Miracloth and incubated 24 hours in 80 ml of a 13 mg/ml solution of NovoZym 234 (Novo Laboratories, Wilton, CT) in 0.7 M NaCl. Protoplast enzyme mixtures were filtered aseptically through four layers of cheesecloth to remove hyphae and other debris, and protoplasts were harvested by centrifugation at 100 ng for 5 min.

Protoplast Fusion. Protoplasts were fused using a procedure similar to that described by Turgeon et al.,

1985, supra. One ml of a suspension containing about 10 protoplasts in STC were prepared with about that contained equal numbers of protoplasts from each parental strain. To this was added 200 $\mu$l of solution containing 60% (w/v) polyethylene glycol (PEG) solution (appx. molecular weight 3350, Sigma Chemical Co., St. Louis, MO), 10mM CaCl$_2$, and 10 mM tris-HCl, pH 7.5 The PEG was mixed with the protoplast suspension by gently rolling the tube. A second 500 $\mu$l aliquot of the PEG solution was added, the mixture again gently mixed, and finally a third 500 $\mu$l aliquot was added and mixed by rolling. Following incubation at 30°C for 10 minutes, the mixture was then diluted with 1.1 ml of STC, the mixture mixed gently. This dilution step was repeated, and finally 2.2 ml of STC were added. After fusion and dilution, protoplasts were recovered by centrifugation and resuspended in 5 ml STC.

Test of Biological Properties. Selected strains were tested for their biocontrol capability, and for their rhizosphere competence. All tests utilized cucumber (Cucumis sativus L) 'Slicemaster' seeds.

Tests of biocontrol capability were conducted by planting treated seeds in Pythium - infested soil. Solid matrix priming (SMP) seed treatments are described elsewhere (Harman and Taylor Phytopathology 78: 520-525 (1988), as is the method of infesting soil with Pythium. In cddition, seeds were also treated using a conventional method slurry technique (Hadar et al., 1984, supra). Tests for biocontrol capability were performed with both SMP and Slurry-treated seeds as described elsewhere (Harman and Taylor, 1988, supra).

Rhizosphere competence was determined by a modification of the method of Chao et al., 1986, supra. Split plastic pipes 22 mm in diameter and 14 cm long were taped together to form a tube, and stoppered on one end with cotton. Noninfested Arkport sandy loam at -7 mPa$_2$ was used to frill the tubes, and was compacted to give a bulk density of about 1.1. One treated seed was planted in each tube, and these were then placed in a humid chamber that maintained 100% relative humidity. After incubation for 4 days, tape was removed from the tubes, the two halves separated to expose the undisturbed soil core, and the seedling lifted from the soil. Roots were gently tapped to remove looselyadhering soil, and the roots were cut into 1 cm sections. These were placed on plates containing a Trichoderma-selective medium (Harman and Taylor, 1988 , supra) and incubated for 3 days.

## RESULTS

Progeny from crosses between T12 and T95 initially grew very slowly, and are described in Example 1. More rapid-growing sectors arose, and these could be divided into several classes. Those growing very slowly were not tested since good growth and proliferation are required for biological control.

Strains tested for biocontrol activity were either with isozyme phenotypes identical to the T95 parent and with rapid growth; with isozyme phenotypes identical to the T12 parent, moderate growth, and antibiotic production; or with isozyme phenotypes like the T12 parent and with very rapid growth. In preliminary tests, only the latter types gave improved biocontrol capability, and three were tested more extensively. These are designated strains 1295-7 (ATCC 20846), 1295-22 (ATCC 20847). and 1295-74 (ATCC 20848).

When applied to cucumber seeds and planted in infested soil, the results shown in Fig. 2 were obtained. When added only in a Methocel slurry, the three progeny strains and the parental T12 and T95 strains are performed similarly. However, strain 1295-22 gave significantly more total seedlings than treatment with any other strain. By 7 days after planting, however, only 6-18% of seedlings survived subsequent attack by Pythium (Fig. 1B).

When these strains were applied in SMP, the progeny strains performed markedly better than the parental strains. Initial stands 3 days after planting were similar with all treatments. Subsequently, however, post-emergence damping off occurred in the seedlings treated with the parental strains, but not with the progeny. Eighty-six to 96% of the seedlings arising from progeny-treated strains survived to the end of the experiment (8 days), while only 64 to 26% of seedlings arising from T12 and T95, respectively, survived to the end of the experiment (Fig. 2A).

Moreover, the seedlings that did survive differed markedly in appearance. Those arising from seeds treated with SMP or alone or with SMP + T12 or T95 had cupped petioles, and appeared unhealthy, while those arising from seeds treated with SMP + any of the progeny strains were vigorous and had normal cotyledons. When removed from soil, the abnormal-appearing seedlings were found to have reduced root systems with browning. Conversely, root systems from the normal-appearing seedlings arising from SMP + progeny-treated seeds were larger and whiter. The reduced root systems with browning are symptomatic of attack by Pythium spp.

When rhizosphere competence was measured, parental and most progeny strains colonized similar number of root sections. Thus, 50, 63, 54 and 59% of root sections were colonized by strains T12, T95, 1295-7, and 1295-74. However, strain 1295-22 colonized 84% of root sections, and appears to possess superior rhizosphere competence.

These results demonstrate that, particularly when applied in SMP, strains 1295-7, 1295-22, and 1295-74 provide nearly perfect stands of cucumbers in Pythium-infected soil. Additionally, SMP + these progeny strains provide excellent protection against damping-off. This is particularly remarkable when the fact that numbers of Pythium propagules are such that 0% stands are obtained with non-treated seeds, and that protection provided by SMP + parental strains is superior to that provided by the fungicide thiram, with or without SMP.

Moreover, these progeny strains + SMP provide excellent protection against seedling disease. This is shown both by the lack of post-emergence damping off, and by the lack of root disease 8 days after planting.

Results with conventional slurry treatment with a Methocel sticker provided fewer differences. This lack of difference may result from the fact that Pythium spp attack seeds very rapidly (Harman and Hadar, 1983, supra), and may infect seeds more rapidly than Trichoderma spores applied in a Methocel slurry can germinate. Thus, many seeds may become irreversably infected before even the best Trichoderma strains become active. When seeds are given SMP, however, Trichoderma spores can germinate and become active before they are planted. Thus superior biocontrol agents may be more easily identified by their superior results with SMP than with a conventional slurry treatment.

Finally, strain 1295-22 appears to be highly rhizosphere competent. The ability of a biocontrol agent to colonize root surfaces permits it to be in the best position to protect roots against attack by soil-borne fungi, and is a valuable attribute.

## Claims
### Claims for the following Contracting States : AT, BE, CH, DE, FR, GB, IT, LI, LU, NL, SE

1. A fused protoplast Trichoderma selected from the group consisting of 1295-7 (ATCC 20846), 1295-74 (ATCC 20847), and 1295-22 (ATCC 20848).

2. The fused protoplast Trichoderma as is claim 1 which is 1295-22 (ATCC 20848).

3. A method of protecting seeds against soilborne pathogens susceptible to attack by Trichoderma spp. which comprises coating the seeds with a soilborne pathogen attack inhibiting amount of a fused protoplast Trichoderma as in claim 1.

4. A method of osmoconditioning seeds and protecting them against soilborne pathogens which comprises admixing (a) seeds coated with a fused protoplast Trichoderma as in claim 1, (b) a particulate solid matrix material and (c) a seed osmoconditioning amount of water, for a time and at a temperature sufficient to cause the seeds to imbibe sufficient water to enhance resultant plant vigor but insufficient to cause seed sprouting; said mixture having a water potential $\psi$ at equilibrium between about -0.5 to about -2.0 megapascals; said particulate solid matrix material being nonpathogenic to the seeds and being friable when admixed with (c) so that it can be machanically separated from the seeds after the treatment without harm to the seeds.

5. The method of claim 4 where the solid matrix material is a carbonaceous material.

6. The method of claim 5 where the carbonaceous material is a lignateous material.

7. The method of claim 6 where the lignateous material is a coal or lignateous shale.

8. The method as in claim 4 where the seeds are cucumber, lettuce, carrot, onion, melons, sweet corn, tomatoes, egg plant or peppers.

9. Seeds osmoconditioned by the process of claim 4.

10. Seeds coated with a Trichoderma as in claim 1.

### Claims for the following Contracting States : GR, ES

1. A method of protecting seeds against soilborne pathogens susceptible to attack by Trichoderma spp.

which comprises coating the seeds with a soilborne pathogen attack inhibiting amount of a fused protoplast Trichoderma selected from the group consisting of 1295-7 (ATCC 20846), 1295-74 (ATCC 20847), and 1295-22 (ATCC 20848).

2.  The method of claim 1 wherein the fused protoplast Trichoderma is 1295-22 (ATCC 20848).

3.  A method of osmoconditioning seeds and protecting them against soilborne pathogens which comprises admixing (a) seeds coated with a fused protoplast Trichoderma selected from the group consisting of 1295-7 (ATCC 20846), 1295-74 (ATCC 20847), and 1295-22 (ATCC 20848), (b) a particulate solid matrix material and (c) a seed osmoconditioning amount of water, for a time and at a temperature sufficient to cause the seeds to imbibe sufficient water to enhance resultant plant vigor but insufficient to cause seed sprouting; said mixture having a water potential $\psi$ at equilibrium between about -0.5 to about -2.0 megapascals; said particulate solid matrix material being nonpathogenic to the seeds and being friable when admixed with (c) so that it can be mechanically separated from the seeds after the treatment without harm to the seeds.

4.  The method of claim 3 wherein the fused protoplast Trichoderma is 1295-22 (ATCC 20848).

5.  The method of claim 3 or 4 where the solid matrix material is a carbonaceous material.

6.  The method of claim 5 where the carbonaceous material is a lignateous material.

7.  The method of claim 6 where the lignateous material is a coal or lignateous shale.

8.  The method as in claim 4 where the seeds are cucumber, lettuce, carrot, onion, melons, sweet corn, tomatoes, egg plant or peppers.


**Patentansprüche**
**Patentansprüche für folgende Vertragsstaaten : AT, BE, CH, DE, FR, GB, IT, LI, LU, NL, SE**

1.  Fusionierter Protoplast Trichoderma, ausgewählt aus der Gruppe, bestehend aus 1295-7 (ATCC 20846), 1295-74 (ATCC 20847) und 1295-22 (ATCC 20848).

2.  Fusionierter Protoplast Trichoderma nach Anspruch 1, dadurch **gekennzeichnet,** daß er 1295-22 (ATCC 20848) ist.

3.  Verfahren zum Schutz von Samen gegen pathogene Organismen aus dem Boden, die von Trichoderma spp. angegriffen werden können, dadurch **gekennzeichnet,** daß man die Samen mit einer den Angriff durch einen pathogenen Organismus aus dem Boden hemmenden Menge eines fusionierten Protoplasten Trichoderma nach Anspruch 1 umhüllt.

4.  Verfahren zum Osmokonditionieren von Samen und zu ihrem Schutz gegen pathogene Organismen aus dem Boden, dadurch **gekennzeichnet,** daß man (a) die mit einem fusionierten Protoplasten Trichoderma nach Anspruch 1 umhüllten Samen, (b) ein teilchenförmiges festes Matrixmaterial und (c) eine die Samen osmokonditionierende Menge Wasser für eine Zeit und bei einer Temperatur vermischt, welche ausreichen, die Samen ausreichend Wasser zur Erhöhung der entstehenden Pflanzenvitalität aufnehmen zu lassen, aber nicht ausreichen, um die Samen keimen zu lassen, wobei das Gemisch ein Wasserpotential $\psi$ im Gleichgewicht zwischen etwa -0,5 bis etwa -2,0 Megapascal besitzt, das teilchenförmige feste Matrixmaterial für die Samen nicht pathogen ist und bei Vermischen mit (c) krümelig ist, so daß es mechanisch von den Samen nach der Behandlung ohne Schaden für die Samen abgetrennt werden kann.

5.  Verfahren nach Anspruch 4, dadurch **gekennzeichnet,** daß das Material ein kohlenstoffhaltiges Material ist.

6.  Verfahren nach Anspruch 5, dadurch **gekennzeichnet,** daß das kohlenstoffhaltige Material ein ligninartiges bzw. holzartiges Material ist.

**7.** Verfahren nach Anspruch 6, dadurch **gekennzeichnet,** daß das ligninartige bzw. holzartige Material ein Kohle- oder Ligninschiefer ist.

**8.** Verfahren nach Anspruch 4, dadurch **gekennzeichnet,** daß die Samen Gurke, Kopfsalat, Karotte, Zwiebel, Melonen, Süßmais, Tomaten, Auberginen oder Pfeffer sind.

**9.** Samen, die nach dem verfahren nach Anspruch 4 osmokonditioniert worden sind.

**10.** Samen, die mit Trichoderma nach Anspruch 1 umhüllt worden sind.

**Patentansprüche für folgende Vertragsstaaten : GR, ES**

**1.** Verfahren zum Schutz von Samen gegen pathogene Organismen aus dem Boden, die von Trichoderma spp. angegriffen werden können, dadurch **gekennzeichnet,** daß man die Samen mit einer den Angriff durch einen pathogenen Organismus aus dem Boden inhibierenden Menge eines fusionierten Protoplasten Trichoderma, ausgewählt aus der Gruppe, bestehend aus 1295-7 (ATCC 20846), 1295-74 (ATCC 20847) und 1295-22 (ATCC 20848), umhüllt.

**2.** Verfahren nach Anspruch 1, dadurch **gekennzeichnet,** daß der fusionierte Protoplast Trichoderma 1295-22 (ATCC 20848) ist.

**3.** Verfahren zum Osmokonditionieren von Samen und zu ihrem Schutz gegen pathogene Organismen aus dem Boden, dadurch **gekennzeichnet,** daß man (a) die mit einem fusionierten Protoplasten Trichoderma, ausgewählt aus der Gruppe, bestehend aus 1295-7 (ATCC 20846), 1295-74 (ATCC 20847) und 1295-22 (ATCC 20848), umhüllten Samen, (b) ein teilchenförmiges festes Matrixmaterial und (c) eine die Samen osmokonditionierende Menge Wasser für eine Zeit und bei einer Temperatur vermischt, welche ausreichen, die Samen ausreichend Wasser zur Erhöhung der entstehenden Pflanzenvitalität aufnehmen zu lassen, aber nicht ausreichen, um die Samen keimen zu lassen, wobei das Gemisch ein Wasserpotential $\psi$ im Gleichgewicht zwischen etwa -0,5 bis etwa -2,0 Megapascal besitzt, das teilchenförmige feste Matrixmaterial für die Samen nicht pathogen ist und bei Vermischen mit (c) krümelig ist, so daß es mechanisch von den Samen nach der Behandlung ohne Schaden für die Samen abgetrennt werden kann.

**4.** Verfahren nach Anspruch 3, dadurch **gekennzeichnet,** daß der fusionierte Protoplast Trichoderma 1295-22 (ATCC 20848) ist.

**5.** Verfahren nach Anspruch 3 oder 4, dadurch **gekennzeichnet,** daß das feste Matrixmaterial ein kohlenstoffhaltiges Material ist.

**6.** Verfahren nach Anspruch 5, dadurch **gekennzeichnet,** daß das kohlenstoffhaltige Material ein lignin- bzw. holzartiges Material ist.

**7.** Verfahren nach Anspruch 6, dadurch **gekennzeichnet,** daß das lignin- bzw. holzartige Material ein Kohle- oder Ligninschiefer ist.

**8.** Verfahren nach Anspruch 4, dadurch **gekennzeichnet,** daß die Samen Gurke, Kopfsalat, Karotte, Zwiebel, Melonen, Süßmais, Tomaten, Auberginen oder Pfeffer sind.

**Revendications**

**Revendications pour les Etats contractants suivants : AT, BE, CH, DE, FR, GB, IT, LI, LU, NL, SE**

**1.** Protoplaste fusionné de Trichoderma choisi dans le groupe consistant en 1295-7 [ATCC 20846], 1295-74 [ATCC 20847] et 1295-22 [ATCC 20848].

**2.** Protoplate fusionné de Trichoderma suivant la revendication 1, qui est le 1295-22 [ATCC 20848].

**3.** Procédé pour protéger der des graines contre des pathogènes du sol sensibles à une attaque par un Trichoderma spp, qui comprend le recouvrement des graines avec une quantité inhibant l'attaque par

EP 0 285 987 B1

les pathogènes du sol d'un protoplaste fusionné de Irichoderma suivant la revendication 1.

**4.** Procédé pour osmoconditioner les graines et les protéger contre des pathogènes du sol, qui comprend le mélange (a) de graines recouvertes d'un protoplaste fusionné de Trichoderma suivant la revendication 1, (b) d'un matériau de matrice solide particulaire et (c) d'une quantité d'eau osmoconditionnant les graines, pendant une période et à une température suffisantes pour que les graines s'imbibent d'une quantité d'eau suffisante pour améliorer la vigueur des plantes résultantes, mais insuffisante pour provoquer une germination des graines; ce mélange ayant un potentiel d'eau $\psi$ a' l'équilibre compris entre environ -0,5 et environ -2,0 mégapascals; ce matériau de matrice solide particulaire étant non pathogène pour les graines et étant friable lorsqu'il est mélangé avec (c) de façon à pouvoir être séparé mécaniquement des graines après le traitement sans dégât pour les graines.

**5.** Procédé suivant la revendication 4, dans lequel le matériau de matrice solide est un matériau carboné.

**6.** Procédé suivant la revendication 5, dans lequel le matériau carboné est un matériau ligneux.

**7.** Procédé suivant la revendication 6, dans lequel le matériau carboné est un charbon ou un schiste ligneux.

**8.** Procédé suivant la revendication 4, dans lequel les graines sont de concombre, laitue carotte, oignon, melon, maïs doux, tomate, aubergine ou poivron.

**9.** Graines osmoconditionnées suivant le procédé de la revendication 4.

**10.** Graines revêtues d'un Trichoderma suivant la revendication 1.

**Revendications pour les Etats contractants suivants : GR, ES**

**1.** Procédé pour protéger des graines contre des pathogènes du sol sensibles à une attaque par un Trichoderma spp, qui comprend le recouvrement des graines avec une quantité inhibant l'attaque par les pathogènes du sol d'un protoplaste fusionné de Trichoderma choisi dans le groupe consistant en 1295-7 [ATCC 20846], 1295-74 [ATCC 20847] et 1295-22 [ATCC 20848].

**2.** Procédé suivant la revendication 1, dans lequel le protoplaste fusionné de Trichoderma estle 1295-22 [ATCC 20848].

**3.** Procédé pour osmoconditioner les graines et les protéger contre des pathogènes du sol, qui comprend le mélange [a] de graines recouvertes d'un protoplaste fusionné de Trichoderma choisi dans le groupe consistant en 1295-7 [ATCC 20846], 1295-74 [ATCC 20847] et 1295-22 [ATCC 20848], [b] d'un matériau de matrice solide particulaire et [c] d'une quantité d'eau osmoconditionnant les graines, pendant une période et à une température suffisantes pour que les graines s'imbibent d'une quantité d'eau suffisante pour améliorer la vigueur des plantes résultantes, mais insuffisante pour provoquer une germination des graines; ce mélange ayant un potentiel d'eau $\psi$ à l'équilibre compris entre environ -0,5 et environ -2,0 mégapascals; ce matériau de matrice solide particulaire étant non pathogéne pour les graines et étant friable lorsqu'il est mélangé avec [c] de façon à pouvoir être séparé mécaniquement des graines après le traitement sans dégât pour les graines.

**4.** Procédé suivant la revendication 3, dans lequel le protoplaste fusionné de Trichoderma est le 1295-22 [ATCC 20848].

**5.** Procédé suivant les revendications 3 ou 4, dans lequel le matériau de matrice solide est un matériau carboné.

**6.** Procédé suivant la revendication 5, dans lequel le matériau carboné est un matériau ligneux.

**7.** Procédé suivant la revendication 6, dans lequel le matériau carboné est un charbon ou un schiste ligneux.

17

**8.** Procédé suivant la revendication 4, dans lequel les graines sont de concombre, laitue, carotte, oignon, melon, maïs doux, tomate, aubergine ou poivron.

Figure 1

Fig.2